# EUROPEAN PATENT APPLICATION

(11) **EP 1 997 428 A1**
(43) Date of publication of application: **03.12.2008**
(21) Application number: 07737929.5
(22) Date of filing: 07.03.2007
(51) Int. Cl.: A61B 5/11, A61B 5/00, A61B 5/0488

(54) **DIAGNOSIS SYSTEM AND PROGRAM TO BE USED IN THE DIAGNOSIS SYSTEM**

(30) Priority: 22.03.2006 JP 2006078544
(71) Applicant: Konica Minolta Medical & Graphic, Inc., Hino-shi, Tokyo 191-8511 (JP)
(72) Inventor: WADA, Yasunori, Tokyo 163-0512 (JP)
(74) Representative: Roberts, Mark Peter
(86) International application number: PCT/JP2007/054415
(87) International publication number: WO 2007/108322

(57) **Abstract**

It is intended to provide a diagnosis system whereby it can be detected whether or not a patient makes aspiration in practice. In this diagnosis system, aspiration sound data is extracted in a controlling section (29) from the biological sound data of a patient stored in the memory section (31) and then the aspiration frequency during the recording is calculated based on the thus extracted aspiration sound data.

## Description

### FIELD OF THE INVENTION

The present invention relates to a diagnosis system that acquires living body information of a subject so as to make a diagnosis on the subject based on the living body information acquired, and a program to be used in the diagnosis system.

### TECHNICAL BACKGROUND

According to the annual Vital statistics of year 2005 issued by the Ministry of Health, Labour and Welfare, the pneumonia is ranked at fourth position, being sucessive to the Malignant growth, the heart trouble and the cerebrovascular disease, among various kinds of causes of death for the Japanese people, and 90 % of the death toll caused by the pneumonia is occupied by the senior citizens older than 65 years old. Accordingly, almost of all dead people caused by the pneumonia are categorized in the sensor citizen pneumonia.

Although an attack of the senior citizen pneumonia may be caused by an inhalation of pathogenic microbe, a spread of an infectious lesion, a Hematogenous infection, etc., it has been considered that almost of all such the attacks are categorized in the Aspiration-related pneumonia that is caused by the "Aspiration" in which fodds, saliva or the like enters into the trachea, instead of the gullet. Accordingly, in order to reduce a number of people attacked by the senior citizen pneumonia, it is effective to discover a patient who currently suffers from the "Aspiration", in its early stage, so as to apply appropriate treatment and prescription to the patient concerned before the Aspiration-related pneumonia acctually attacks the patient concerned. Further, when there is a possibility that a patient may suffer from the "Aspiration", for heightening the effects of treatments to be applied to the patient concerned, it is important not only to assume an occurrence of the Aspiration-related pneumonia, but also to speedily apply medical inspections for detecting the pneumonia, such as the X ray radiographing operation, etc., to the patients concerned, in order to discover the Aspiration-related pneumonia in its early stage.

Patent Document 1 sets forth a method for detecting whether or not the deglutition reflex of the patient deteriorates, by comparing the deglutition reflex parameters of the patient concerned with another deglutition reflex parameters of a physically unimpaired person, acquired in advance. According to the method set forth in the Patent Document 1, various kinds of sensors, such as an electrode for measuring data of the Electromyograph (EMG), a microphone for detecting sounds of deglutition, an acceleration sensor for detecting bibrations generated at the time of deglutition, are attached or mounted onto the throat of the patient, so as to detects the deglutition reflex parameters of the patient (including a muscle activation time, a muscle activation amount, a duration time from a deglutition sound waveform, a time required for raising the larynx, etc.) when the patient deglutires (to swallows down) foods, and then, the deglutition reflex parameters of the patient, detected in the above, are compared to those of the physically unimpaired person, acquired in advance, so as to detecte whether or not the deglutition reflex of the concerned patient deteriorates to a level lower than the other deglutition reflex of the physically unimpaired person.
[Patent Document 1]
   Tokkai 2005-304890 (Japanese Non-Examined Patent Publication)

### DISCLOSURE OF THE INVENTION

### SUBJECT TO BE SOLVED BY THE INVENTION

However, the method set forth in the abovementioned Patent Document 1 is not such a method that makes it possible to detect whether or not the patient actually suffers from the aspiration, but merely makes it possible to measure the parameters for detecting whether or not the deglutition reflex of the patient concerned deteriorates to a level lower that that of the physically unimpaired person, though the measuring result could be employed as one of the indexes for grasping the tendency whether or not the patient concerned is liable to suffer from the aspiration.

Further, during sleeping hours of the patient, since the deglutition reflex deteriorates associated with the deterioration of the influence of the sympathetic nerve of the patient, the aspiration becomes liable to attack the patient. Neverthless, in the method set forth in the abovementioned Patent Document 1, since the deglutition reflex parameters are acquired on the basis of the patient's actions for deglutiring (swallowing down) foods, there has arisen a problem that only the deglutition reflex during awakening hours of the patient can be detected.

The present invention is achieved in view of the abovementioned subjects. It is an object of the present invention to provide a diagnosis system which makes it possible to detect whether or not the patient actually suffers from the aspiration.

### MEANS FOR SOLVING THE SUBJECT

In order to solve the abovementioned subject, a diagnosis system for detecting whether or not a subject is affected by an aspiration, embodied in the present invention, is characterized by comprising: a living-body information acquiring section to acquire living body information of the subject; a deglutition information extracting section to extract deglutition information from the living body information acquired by the living-body information acquiring section; and a deglutition frequency calculating section to calculate a number of deglutitions, based on the deglutition information extracted by the deglutition information extracting section.

Further, a program to be employed for a diagnosis system that acquires living body information of a subject so as to detect whether or not the subject is affected by an aspiration, embodied in the present invention, is characterized by comprising and making a computer to implement: a deglutition information extracting process for extracting deglutition information from the living body information; and a deglutition frequency calculating process for calculating a number of deglutitions, based on the deglutition information extracted in the deglutition information extracting process.

### EFFECT OF THE INVENTION

According to the present invention, since the number of deglutitions performed by the subject is calculated on the basis of the living body information acquired from the subject concerned, it becomes possible for the doctor to grasp the fact that the number of deglutitions that ought to occur by nature is zero or smaller than usual during the time interval of acquiring the living body information, based on the above-calculated number of deglutitions. This makes it possible to acquire information even in regard to the inapparent aspiration, which has been conventionally regarded as very difficult for the doctor to grasp. Therefore, it becomes possible for the doctor to securely determine the state of the subject, and accordingly, to apply appropriate medical treatement and prescription being appropriate for the current state of the subject concerned.

### BRIEF DESCRIPTION OF THE DRAWINGS

Fig. 1 shows a schematic diagram of a diagnosis system.
Fig. 2 shows an exemplified screen to be displayed on a display section provided in a patient terminal device.
Fig. 3 shows a flowchart for explaining operations to be conducted by a control section.
Fig. 4 shows an exemplified screen to be displayed on a display section provided in a doctor terminal device.
Fig. 5 shows a flowchart for explaining the operations to be conducted by the control section 29.
Fig. 6(a) shows an exemplified analogue waveform of living body sound acquired from a sleeping patient, while
Fig. 6(b) shows an enlarged waveform in a region surrounded by a circular dot line shown in Fig. 6(a).

### EXPLANATION OF THE NOTATIONS

- 1: a diagnosis system
- 3: a microphone
- 12: a patient terminal device
- 13: a doctor terminal device
- 14: a server
- 29: a control section
- 31: a memory section

### BEST MODE FOR IMPLEMENTING THE INVENTION

Referring to the drawings, a preferred embodiment of the present invention will be detailed in the following. In this connection, the technical scope of the present invention is not limited to the technical terms and/or the decisive descriptions to be employed for describing the preferred embodiment of the present invention.

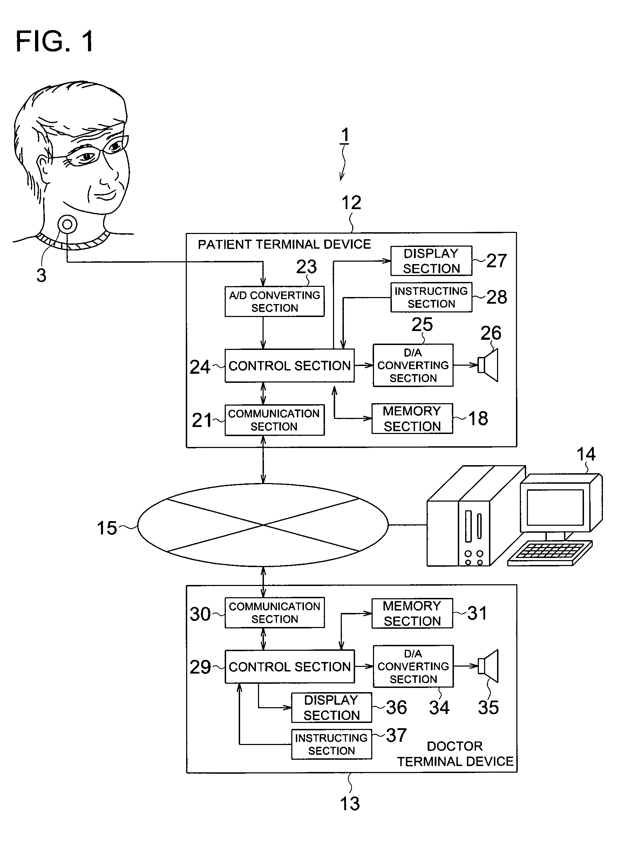

Fig. 1 shows a configuration of a diagnosis system 1 embodied in the present invention. As shown in Fig. 1, the diagnosis system 1 is constituted by a microphone 3 (serving as a living body information acquiring section and a cough information extracting section in the claimed present invention), a patient terminal device 12, a doctor terminal device 13, a server 14 and a communication network 15.

The patient terminal device 12 is mainly installed into a house of patient, so as to make it possible to apply a predetermined processing to living body sound data (serving as the living body information) pick-upped and transmitted by the microphone 3 and to store the processed data into the server 14, and further, to output the deglutition sounds from a speaker 26.

The doctor terminal device 13 is mainly installed into a hospital, etc., so as to make it possible to determine whether or not the concerned patient suffers from the aspiration by reading out the living body information stored in the server 14 and parsing the read data.

The server 14 is provided with a storage device that is capable of storing massive data, and is installed into the hospital in which the doctor terminal device 13 is also installed, or a data processing center facility or the like. The server 14 stores deglutition sound data (deglutition information) of a physically unimpaired person and/or that of a physically impaired person (a patient who suffers from aspiration), both acquired in advance, into a database.

The patient terminal device 12 and the doctor terminal device 13 are coupled to the server 14 through the communication network 15, so as to form a network system. It is desirable that the communication network 15 is the Internet. In this connection, although the single patient terminal device 12 and the single doctor terminal device 13 are respectively coupled to the server 14 in the present embodiment in order to simplify the explanation of the system, it is needless to say that the present embodiment can be easily expanded to such a system that includes a plurality of patient terminal devices 12 and a plurality of doctor terminal devices 13.

The patient terminal device 12 is constituted by a memory section 18, a communication section 21, an analogue-to-digital converting section 23 (hereinafter, referred to as a A/D converting section 23), a digital-to-analogue converting section 25 (hereinafter, referred to as a D/A converting section 25), a speaker 26, a display section 27, an instructing section 28 and a control section 24 to control the various kinds of sections mentioned in the above. Further, the microphone 3 is coupled to the patient terminal device 12 through a pin jack, being audio use. The microphone 3 is attached onto a patient body at a position to be diagnosed (for instance, a chin or a throat) by using an adhesive material, so as to make it possible to acquire the living body sounds of the patient by employing a sound collecting section (not shown in the drawings) provided in the microphone 3.

The memory section 18 is constituted by a RAM (Random Access Memory), a ROM (Read Only Memory), an HDD (Hard Disc Drive), etc. Since the memory section 18 temporarily stores data, necessary for processing operations to be performed in each of the constituent sections of the patient terminal device 12, therein, it is possible to make the patient terminal device 12 operate speedily and stably. In addition, the memory section 18 stores the living body sound data transmitted from the microphone 3 and programs to be executed for operating the control section 24, therein.

The communication section 21 is so constituted that the patient terminal device 12 can conduct operations for communicating information with the server 14 through the communication network 15 by employing a wired or wireless communicating device. Concretely speaking, the patient terminal device 12 transmits the living body sound data, serving as digital data converted from analogue sound signals, to the server 14 through the communication section 21.

The A/D converting section 23 converts the analogue sound signals, representing the living body sounds and outputted by the microphone 3, to the living body sound data. Then, the living body sound data is inputted into the control section 24.

The control section 24 is provided with a CPU (Central Processing Unit), so as to control each of the constituent sections provided in the patient terminal device 12, by executing the programs stored in the memory section 18. For instance, the control section 24 outputs the various kinds of digital data, inputted from the A/D converting section 23 as needed, to the memory section 18, and transmits digital data to the server 14 on the basis of the instructions inputted from the instructing section 28.

The speaker 26 outputs audible sounds based on the analogue sound signals converted from the digital sound data by the D/A converting section 25.

The display section 27 is constituted by a display device, such as a CRT (Cathode Ray Tube), a LCD (Liquid Crystal Display), an Organic Electro-luminescence, a plasma display, a projection device, etc., so as to make it possible to display waveforms of the analogue sound signals outputted by the microphone 3, status information of each of the constituent sections, etc.

The instructing section 28 is constituted by a keyboard, a mouse, a tracking ball, a touch panel, etc., so as to make it possible for the patient or a helper, closely contacting with the patient, to input various kinds of instructions while viewing the display section 27.

When the instruction for outputting audible sound of the living body sound is inputted from the instructing section 28, the control section 24 outputs the living body sound data stored in the memory section 18 to the D/A converting section 25. Then, the D/A converting section 25 converts the digital sound data to the analogue sound signals, and outputs the analogue sound signals to the speaker 26, so that the speaker 26 generates the audible sounds based on the analogue sound signals outputted by the D/A converting section 25. According to the abovementioned process, it is possible for the patient terminal device 12 to reproduce the living body sound of the patient, picked up in advance by employing the microphone 3, as needed. As a result of hearing the reproduced living body sound, either the patient or the helper determines whether or not the living body sound concerned should be registered into the server 14, and operates the instructing section 28 to instruct the patient terminal device 12 about the determining result.

When the control section 24 registers the digital sound data, stored in the memory section 18, into the server 14, based on the instruction inputted by the patient or the like, the control section 24 makes the communication section 21 transmit the concerned data to the server 14 through the communication network 15, so that the server 14 can store the concerned data therein.

The doctor terminal device 13 is constituted by a communication section 30, a memory section 31, a D/A converting section 34, a speaker 35, a display section 36, an instructing section 37 and a control section 29 to control the various kinds of sections mentioned in the above.

The communication section 30 is so constituted that the doctor terminal device 13 can conduct operations for communicating information with the server 14 through the communication network 15 by employing a wired or wireless communicating device. Concretely speaking, the communication section 30 downloads the living body sound data, stored in the server 14, from the server 14 through the communication section 30.

The memory section 31 is constituted by a RAM, a ROM, an HDD, etc., so that the memory section 31 stores the living body sound data downloaded from the server 14 through the communication section 30 and diagnosis programs to be executed for making diagnosis (not shown in the drawings), therein.

The speaker 35 outputs audible sounds based on the analogue sound signals converted from the digital sound data by the D/A converting section 34. The sounds to be generated by the speaker 35 are the living body sounds of the patient, picked up by the microphone 3, and are the same as those outputted by the speaker 26 of the patient terminal device 12.

The control section 29 is provided with a CPU (Central Processing Unit), so as to control each of the constituent sections provided in the doctor terminal device 13, by executing the programs stored in the memory section 31. For instance, the control section 29 controls the communication section 30 to download the digital data from the server 14 and stores the downloaded digital data into the memory section 31, and then, conducts the controlling operation for outputting the audible sound based on the downloaded digital data from the speaker 35, according to the instruction inputted from the instructing section 37. In this connection, the control section 29 also serves as a deglutition information extracting section and an deglutition number calculating section and a determining section, described in the claimed present invention.

The display section 36 is constituted by a display device, such as a CRT (Cathode Ray Tube), a LCD (Liquid Crystal Display), an Organic Electro-luminescence, a plasma display, a projection device, etc., so as to make it possible not only to display waveforms of the analogue sound signals, converted from the digital sound data stored in the memory section 31 by a D/A converting section (not shown in the drawings), but also to display status information of each of the constituent sections of the doctor terminal device 13 and other information, thereon.

The instructing section 37 is constituted by a keyboard, a mouse, a tracking ball, a touch panel, etc., so as to make it possible for the doctor, a nurse or the like to input various kinds of instructions while viewing the display section 36. The doctor terminal device 13 is so constituted that, in response to the instruction inputted from the instructing section 37 by the doctor, the nurse or the like, the control section 29 transmits the living body sound data, stored in the memory section 31, to the D/A converting section 34, and then, the speaker 35 outputs the audible sound based on the analogue sound signals converted by the D/A converting section 34. In other words, by operating the instructing section 37, the doctor, the nurse or the like can repeatedly hear the living body sounds of the concerned patient at any time.

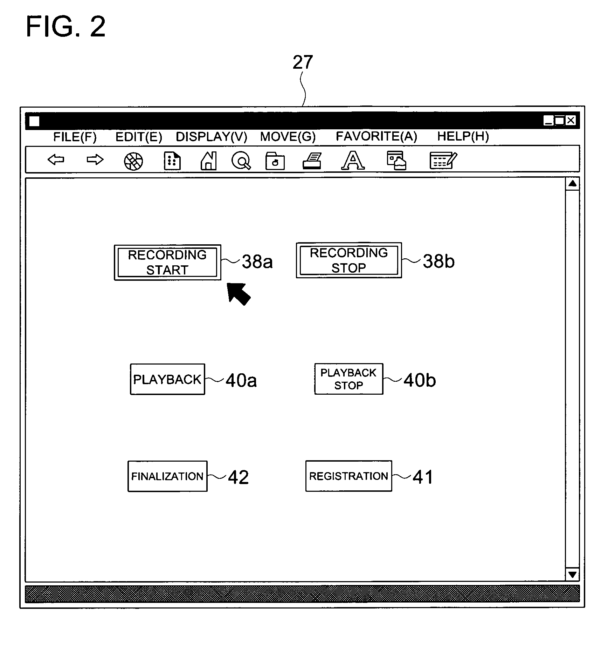

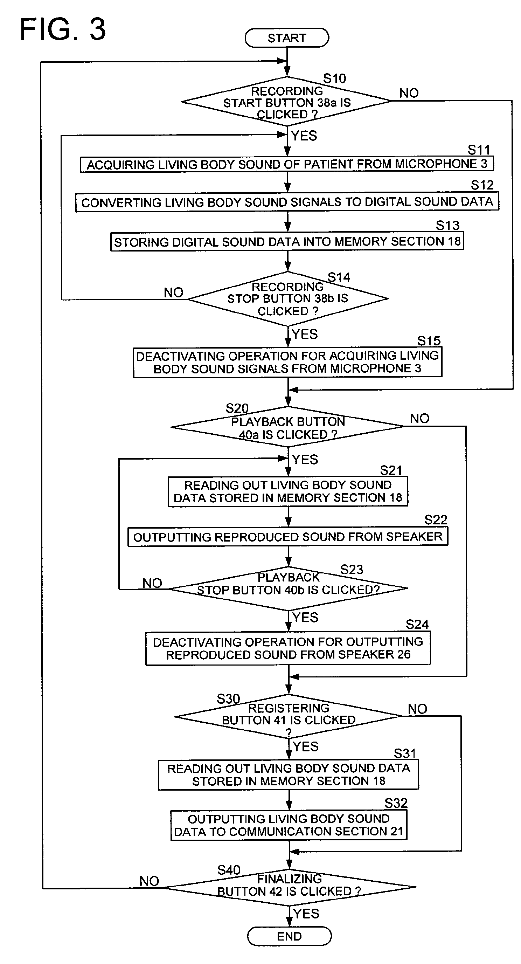

Next, referring to Fig. 2 and Fig. 3, actions of the patient terminal device 12 will be detailed in the following.

Fig. 2 shows an exemplified screen to be displayed on the display section 27 provided in the patient terminal device 12. On the screen, a recording start button 38a, a recording stop button 38b, a playback button 40a, a playback stop button 40b, a registering button 41 and a finalizing button 42 are displayed.

Fig. 3 shows a flowchart for explaining the operations to be conducted by the control section 24. The flowchart shown in Fig. 3 starts at the time when the patient terminal device 12 enters into such a state that the microphone 3 is attached onto a patient body at a position to be diagnosed by using an adhesive material, and the various kinds of buttons are displayed on the display section 27 as shown in Fig. 3. In this connection, considering that the aspiration is liable to occur during the sleeping time in which the influence of the sympathetic nerve is deteriorated to a lower level, an example of making a diagnosis by calculating a number of patient's deglutitions occurring during the sleeping time will be detailed as the preferred embodiment of the present invention in the following.

The control section 24 always monitors whether or not the recording start button 38a is clicked (Step S10), whether or not the playback button 40a is clicked (Step S20), whether or not the registering button 41 is clicked (Step S30) and whether or not the finalizing button 42 is clicked (Step S40). In this connection, initially at most, since no operations other than the recording start operation is performed, the patient terminal device 12 may be configured in such a manner that no buttons other than the recording start button 38a can be clicked.

When determining that the recording start button 38a is clicked (Step S10; YES), the control section 24 acquires the living body sound of the patient concerned as the analogue sound signals by using the microphone 3 (Step S11), and then, makes the A/D converting section 23 convert each of the analogue sample levels of the analogue sound signals to digital level data for every 50 microsecond (Step S12). Generally speaking, although the shorter the sampling period to be employed in the A/D converting section 23 becomes, the more accurately the recording operation can be performed and the sound can be reproduced, an average person can hear the sound, reproduced from such the discrete digital sound data that is converted from the analogue sound signals by setting the sampling frequency even at 20 kHz, as a normally reproduced analogue sound. Accordingly, in the present embodiment, the sampling period is set at 50 microseconds. Successively, the control section 24 makes the memory section 18 store the digital sound data converted from the analogue sound signals therein (Step S13). Every time when repeating the operations from Steps S11 through S13, the control section 24 monitors whether or not the recording stop button 38b is clicked by using the instructing section 28 (Step S14). When determining that the recording stop button 38b is clicked (Step S14; YES), the control section 24 deactivates the operation for acquiring the living body sound signals picked up from the microphone 3 (Step S15).

Successively, when determining that the playback button 40a is clicked (Step S20; YES), the control section 24 reads out the living body sound data stored in the memory section 18 in Step S13 (Step S21), and makes the D/A converting section 25 convert the living body sound data to the reproduced analogue sound signals, so as to make the speaker 26 outputs the reproduced sound (Step S22). Hearing the reproduced sound outputted from the speaker 26, the patient or the helper confirms whether or not the living body sounds of the patient are collected appropriately. Still successively, as a result of monitoring whether or not the playback stop button 40b is clicked by using the instructing section 28 (Step S23), when determining that the playback stop button 40b is clicked (Step S23; YES), the control section 24 deactivates the operation for outputting the reproduced sound from the speaker 26 (Step S24). Then, if no problem happens to the reproduced sound, the control section 24 registers this sound into the server 14. If the living body sounds are not collected successfully due to the influence of noises, etc., it is possible to resume the sound collecting operation by again clicking the recording start button 38a.

When determining that the registering button 41 is clicked by using the instructing section 28 (Step S30; YES), the control section 24 reads out the living body sound data stored in the memory section 18 in Step S13 (Step S31), and outputs the living body sound data to the communication section 21 (Step S32). Then, the communication section 21 transmits the living body sound data to the server 14 through the communication network 15, so that the living body sound data is registered (stored) into the server 14. At the same time, various kinds of other information, such as the patient name allotted in advance to the patient terminal device 12 concerned, the date of recording the living body sound, the recording start time and the recording stop time (recording date information), etc., are also registered (stored) into the server 14.

Successively, when determining that the finalizing button 42 is clicked by using the instructing section 28 (Step S40; YES), the control section 24 finalizes this program.

Since the patient terminal device 12 is operated as described in the foregoing, it becomes possible for the patient, the helper or the like, not only to collect the living body sound at any time on their own convenience, but also to register the collected sound data into the server 14 after confirming whether or not the sound data are collected appropriately.

In this connection, it is applicable that the patient or the like sets a timer in advance, so that the recording start operation and/or the recording stop operation are actually conducted on the basis of the timer. Further, it is also applicable that the system is so constituted that, when the recording operation is deactivated, the patient terminal device 12 automatically outputs the living body sound data to the communication section 21 so as to register them into the server 14. By configuring the system as mentioned in the above, it becomes possible for the patient, the helper or the like to collect the living body sounds and to register them into the server 14 without conducting cumbersome operations.

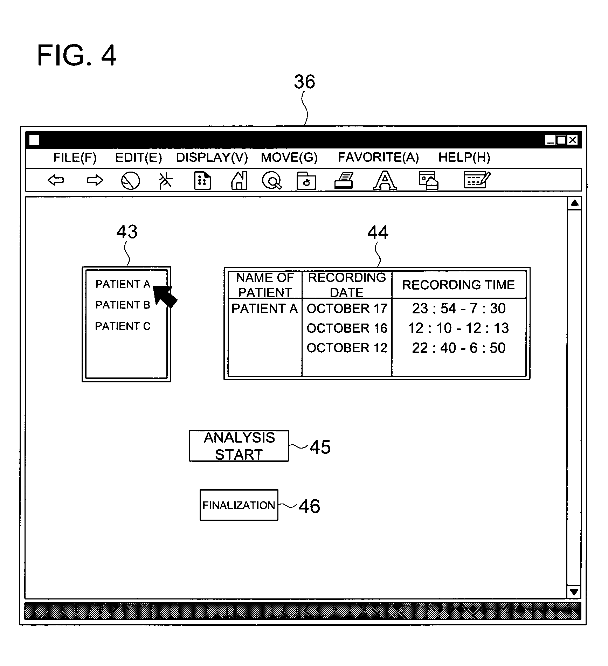

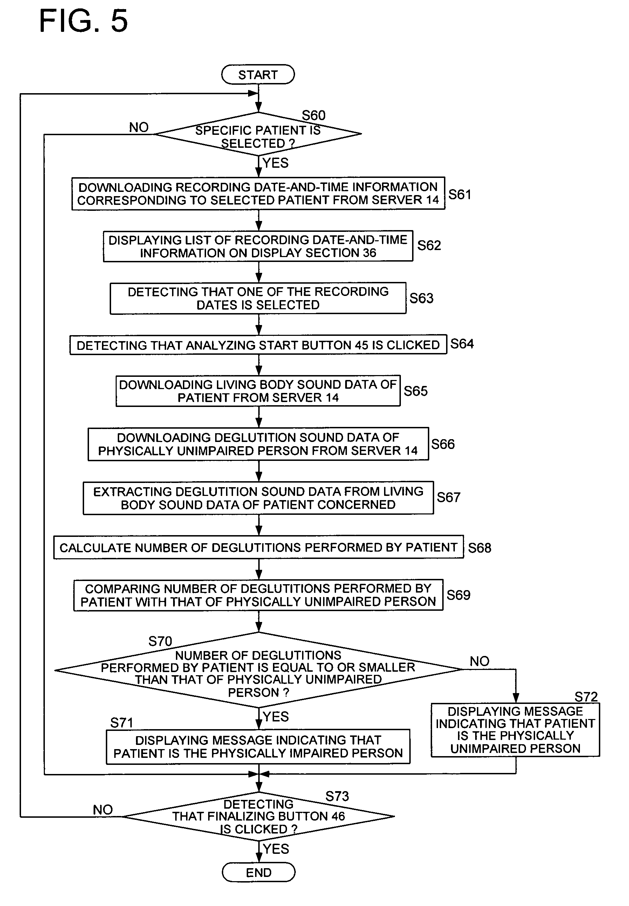

Next, referring to Fig. 4 and Fig. 5, actions of the doctor terminal device 13 will be detailed in the following.

Fig. 4 shows an exemplified screen to be displayed on the display section 36 provided in the doctor terminal device 13. On the screen, there are displayed: a patient selecting box 43 in which names of the patients registered in the server 14 are indicated as a list; a recording date selecting box 44 in which the recording start times and the recording stop times of the living body sound data, registered in the server 14 in respect to the patient selected by using the patient selecting box 43, are indicated as a list; an analyzing start button 45 to start the analyzing operation of the living body sound data selected; and a finalizing button 46 to finalize the program.

Fig. 5 shows a flowchart for explaining the operations to be conducted by the control section 29. The flowchart shown in Fig. 5 starts at the time when the doctor terminal device 13 enters into such a state that the various kinds of boxes and buttons are displayed on the display section 36 as shown in Fig. 4. In this connection, since the doctor terminal device 13 is so constituted that the control section 29 periodically accesses the server 14 at predetermined time intervals, so as to make the communication section 30 download new data from the server 14 through the communication network 15, the contents of the patient selecting box 43 are automatically revised to its latest version. Further, it is assumed that the program, to be employed for conducting the operations indicated in this flowchart, is stored in advance in the memory section 31.

The control section 29 always monitors whether or not a specific patient is selected from the patient selecting box 43 (Step S60) and whether or not the finalizing button 46 is clicked (Step S73).

When determining that one of the patients displayed on the patient selecting box 43 (for instance, a patient A) is selected by using the instructing section 37 (Step S60; YES), the control section 29 accesses the server 14 so as to download the recording date information of the living body sound data corresponding to the patient A (Step S61). Then, the control section 29 controls the display section 36 to display the list of the recording date information of the patient A in the recording date selecting box 44 (Step S62).

Successively, when detecting that one of the recording dates is selected from the list of the recording date information by using the instructing section 37 (Step S63), and still successively, when detecting that the analyzing start button 45 is clicked (Step S64), the control section 29 downloads the living body sound data, corresponding to the recording date selected in the above, from the server 14 and makes the memory section 31 store them therein (Step S65). At the same time the control section 29 also downloads the deglutition sound data of the physically unimpaired person from the server 14 and makes the memory section 31 store them therein (Step S66).

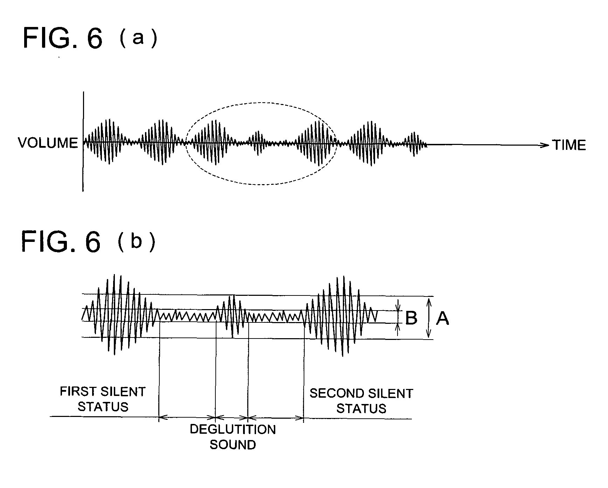

Still successively, the control section 29 extracts the deglutition sound data from the living body sound data of the patient concerned, stored in the memory section 31 (Step S67), in order to calculate a number of deglutitions performed by the patient during the recording state, based on the deglutition sound data extracted in the above (Step S68). As the method for calculating the abovementioned number of deglutitions, by extracting a continuous signal, which lasts a time interval equal to or longer than a predetermined time interval while representing a sound volume equal to or smaller than the maximum sound volume of a general deglutition, as the deglutition, and by totally summing the extracted deglutitions, the number of deglutitions can be calculated. Referring to Fig. 6(a) and Fig. 6(b), the process of the abovementioned calculation will be detailed in the following.

Fig. 6(a) shows an exemplified analogue waveform of the living body sound data acquired from a sleeping patient, while Fig. 6(b) shows an enlarged waveform in a region surrounded by the circular dot line shown in Fig. 6(a). Generally speaking, among various actins that are performed by the sleeping patient, each of a breathing action, an unconscious talking action, a saliva deglutiring action and a teeth grinding action generates a kind of sound. As shown in Fig. 6(b), there can be recognized a characteristic that the amplitude of the sound waveform generated at the time of the saliva deglutiring action is relatively small compared to those of the other actions. Therefore, when the maximum amplitude of the sound waveform, generated at the time of the saliva deglutiring action, is defined as an amplitude A, it is possible to determine the portion of the sound signal, the waveform of which has the amplitude lower than the amplitude A and the duration time of which is equal to or greater than the predetermined time interval, as the deglutition sound.

Further, since the deglutiring action is such a consecutive motion that includes a process for feeding foods from the oral cavity to the pharynx (oral cavity phase), a motion for feeding the foods from the pharynx to the esophagus (pharynx phase) and a process for feeding the foods from the esophagus to the stomach (esophagus phase), the other action, such as the breathing action, the unconscious talking action, the teeth grinding action, etc., is halted during the deglutiring action. As a result, substantially a silent status would last a certain predetermined time interval before and after the occurrence of the deglutition sound. Accordingly, in order to detect the deglutiring action more precisely, when the maximum amplitude of the sound waveform, remaining at the time of the silent status, is defined as an amplitude B, it is applicable that, when the sound signal, the waveform of which has the amplitude equal to or lower than the amplitude B, lasts a time interval equal to or longer than the predetermined time interval (first silent status), and then, the portion of the sound signal, the waveform of which has the amplitude equal to or higher than the amplitude B and equal to or lower than the amplitude A and the duration time of which is equal to or greater than the predetermined time interval, emerges, and successively, the sound signal, the waveform of which has the amplitude equal to or lower than the amplitude B, again lasts a time interval equal to or longer than the predetermined time interval (second silent status), the middle portion of the sound signal is determined as the deglutition sound.

Alternatively, it is also possible that the features of the waveform of the deglutition sound are stored in advance, so as to determine a portion of the detected sound signal, features of which are same as or approximate to those of the waveform stored in advance, as the deglutition sound. In this connection, it is applicable that the system is so constituted that the abovementioned various kinds of data, such as the amplitude A representing a maximum sound level of the deglutition sound, the amplitude B representing a maximum sound level at the time of the silent status, the features of the waveform of the deglutition sound signal, etc., are stored in advance into either the memory section 31 of the doctor terminal device 13 or the server 14, so that the control section 29 can appropriately use each of the abovementioned data as needed.

By totally summing the deglutitions extracted according to the abovementioned process, the control section 29 calculates the number of deglutitions performed during the recording mode by the patient concerned. Then, the control section 29 compares the number of deglutitions acquired from the patient with that of the physically unimpaired person (Step S69).

Generally speaking, as the normal physiological function, there are performed in the human body not only the activities for secreting the various secretions, such as the saliva, etc., but also the deglutition activity for conveying the saliva inside the body. Accordingly, it can be considered that, within a certain time period, the secretion amount of the saliva secreted by the physically unimpaired person is the same as that secreted by the physically impaired person. Therefore, if the number of deglutitions performed by the patient is smaller than that of the physically unimpaired person, it can be determined that the patient swallows the saliva without performing the deglutiring action, namely, the aspiration attacks the patient concerned.

In this connection, even for the physically unimpaired person, the secretion amount of the saliva and/or the degree of the number of deglutitions varies in a certain distributing range. Accordingly, it is desirable that the judgment of whether or not the subject is affected by the aspiration is made by taking the abovementioned distributing range of the physically unimpaired person into account. In other words, it is desirable to employ a statistic index, for instance, such as the standard deviation, etc. Further, it is more desirable to establish in advance a threshold value for determining whether the concerned person is the physically unimpaired person or the physically impaired person, so as to compare the above-established threshold value with the number of deglutitions performed by the patient. According to this method, it becomes possible to easily judge the current status of the patient concerned.

Successively, the control section 29 compares the number of deglutitions performed by the patient with that of the physically unimpaired person in Step S69. When determining that the number of deglutitions performed by the patient is smaller than that of the physically unimpaired person (Step S70; YES), the control section 29 controls the display section 36 to display a message indicating that the aspiration attacks the patient concerned, namely, the patient is the physically impaired person (Step S71). On the other hand, when determining that the number of deglutitions performed by the patient is substantially the same as that of the physically unimpaired person (Step S70; NO), the control section 29 controls the display section 36 to display a message indicating that the aspiration does not attack the patient concerned, namely, the patient is the physically unimpaired person (Step S72).

Still successively, when detecting that the finalizing button 46 is clicked by using the instructing section 37 (Step S73; YES), the control section 29 finalizes this program (END).

In this connection, it is also applicable that the doctor terminal device 13 is so constituted that the control section 29 controls the display section 36 to display the playback button shown in Fig. 4, so as to download the digital sound data from the server 14 when detecting that the playback button is clicked by using the instructing section 37, and then, controls the D/A converting section 34 to convert the downloaded digital sound data to the analogue sound signals based on which the speaker 35 outputs the audible sound. According to the abovementioned configuration, it becomes possible for the doctor to hear the sound of the deglutiring action performed by the patient before analyzing the digital sound data, resulting in a prevention of such an operating error that the doctor erroneously analyzes the other digital sound data by mistake.

Further, in the present embodiment, it is still more desirable to grasp the coughing state of the patient, based on the digital sound data of the patient concerned. It is important from the diagnosis point of view to measure the deglutition information and the cough information together in respect to the patient concerned, and the present invention makes it possible. The state that it is uncertain whether or not the aspiration actually attacks the patient is called the "inapparent aspiration". The term of the "inapparent aspiration" is originated from the fact that it is impossible to clearly detect the occurrence of the aspiration, since the occurrence of the aspiration cannot be apparently recognized if the cough does not attack the patient just after the occurrence of the aspiration, though the occurrence of the aspiration can be apparently recognized if the cough attacks the patient just after the occurrence of the aspiration. Specifically, the aspiration that attacked the patient during midnight is liable not to be recognized, but becomes the "inapparent aspiration", even if the cough attacked the patient, but if the attacked cough was a slight one.

On the other hand, although it is difficult to determine whether or not the aspiration actually attacks the patient, only based on the fact that the number of cough performed during midnight by the patient is relatively greate, the probability that the aspiration actually attacks the patient is relatively high when the number of deglutitions occuring within the measuring period is smaller than that in the normal state, and when the coughs occur at that time, it is indicated that the cough reflection works to some extent. Further, if the cough is detected continuously just after the deglutition is detected, it would be suggested that it is prematured though the deglutition is performed and the cough reflection still works. Accordingly, it is very useful to employ the cough information together with the deglutition information, since the information in regard to both the deglutition function and the cough reflection function can be acquired by using both of them. In this connection, in order to detect the abovementioned cough performed by the patient, it is applicable that the characteristics of the waveform of the cough sound are stored in advance into the memory section 31 of the doctor terminal device 13 or the server 14, so as to determine the detected sound, having the waveform same as or approximate to that stored in advance, as the cough sound.

Further, if no abnormality exists in the saliva secreting function, the saliva is secreted even during a sleeping. In this case, the physically unimpaired person deglutires the saliva secreted, while the physically impaired person (inapparent aspiration patient) washes the secreted saliva down into the lung. Accordingly, if a certain abnormality exists in the saliva secreting function, the number of deglutitions to be inherently performed would also change. Therefore, it is desirable that the saliva secreting capability and the saliva secreting state of the subject are taken into account as the method for improving the accuracy of the diagnosis embodied in the present invention. Concretely speaking, it is possible to employ information, such as the salivary gland disorder, etc., as a parameter when comparing the numbers of deglutitions with each other. For instance, the rest time saliva amount, known as the saliva secreting inspection, can be employed as the useful parameter.

Still further, it is desirable that the operation for acquiring the living body sound data is conducted during the rest time of the patient concerned. It is more desirable that the acquired living body sound data includes the data of the sleeping stage. It has been well known that the reflecting action, such as the deglutition reflection, the cough reflection, etc., relates to the sleeping stage, such as the REM (Rapid Eye Movement) sleep, the non-REM sleep, etc. Accordingly, in order to grasp the current status of the subject, it is very important to grasp that on what sleeping stage the acquired living body sound data is actually acquired. For this purpose, it is desirable that both the acquisition of the living body sound data and the grasp of the sleeping stage are used together in the embodiment of the present invention. In this connection, it is possible to employ the publicly well-known measuring methods, such as an electroencephalography, an electromyography, an ocular movement inspection, etc., as the method for grasping the sleeping stage of the patient.

Still further, although the digital sound data of the patient is compared with that of the physically unimpaired person so as to determine whether or not the aspiration attacks the patient concerned when the digital sound data of the patient is analyzed in the present embodiment aforementioned, it is also applicable that the server 14 is made to store digital sound data of a physically impaired person (patient who is currently suffered from the aspiration) in advance, so that the status of the patient concerned can be determined by comparing the digital sound data of the patient concerned and that of the physically impaired person with each other. Further, it is also applicable that the doctor terminal device 13 is so constituted that an artificial neural network is made to learn data of the patient, diagnosis on which was made in the past, so as to make good use of the past data for the next diagnosis.

Yet further, the scope of the device to be used for acquiring the living body information of the patient is not limited to the microphone. For instance, it is also applicable that an acceleration detecting sensor is disposed on the throat of the patient, so that the acceleration detecting sensor detects the movements of the throat so as to acquire the living body information of the patient, or alternatively, electrodes are disposed on the throat of the patient or at the positions in the vicinity of the throat, so that the living body information of the patient can be acquired by detecting the micro-electric current flowing between the electrodes at the time of the muscular contraction of the throat.

## Claims

1. A diagnosis system for detecting whether or not a subject is affected by an aspiration, **characterized by** comprising:
a living-body information acquiring section to acquire living body information of the subject;
a deglutition information extracting section to extract deglutition information from the living body information acquired by the living-body information acquiring section; and
a deglutition frequency calculating section to calculate a number of deglutitions, based on the deglutition information extracted by the deglutition information extracting section.

2. The diagnosis system recited in claim 1, **characterized by** further comprising:
a determining section to determine whether or not the subject is affected by the aspiration, by comparing at least one of a number of deglutitions of a physically unimpaired person and a number of deglutitions of a physically impaired person, both acquired in advance, with the number of deglutitions calculated by the deglutition frequency calculating section.

3. The diagnosis system recited in claim 1 or 2,
**characterized in that**
the living-body information acquiring section is selected from at least one of a microphone, an acceleration detecting sensor and an electrode.

4. The diagnosis system recited in any one of claims 1-3, **characterized by** further comprising:
a cough information extracting section to extract cough information of the subject from the living body information acquired by the living-body information acquiring section.

5. A program to be employed for a diagnosis system that acquires living body information of a subject so as to detect whether or not the subject is affected by an aspiration, **characterized by** comprising and making a computer to implement:
a deglutition information extracting process for extracting deglutition information from the living body information; and
a deglutition frequency calculating process for calculating a number of deglutitions, based on the deglutition information extracted in the deglutition information extracting process.

6. The program to be employed for the diagnosis system recited in claim 5, **characterized by** further comprising and implementing:
a determining process for determining whether or not the subject is affected by the aspiration, by comparing at least one of a number of deglutitions of a physically unimpaired person and a number of deglutitions of a physically impaired person, both acquired in advance, with the number of deglutitions calculated in the deglutition frequency calculating process.

7. The program to be employed for the diagnosis system recited in claim 5 or 6, **characterized by** further comprising and implementing:
a cough information extracting process for extracting cough information from the living body information acquired in the living-body information acquiring process.
